# EUROPEAN PATENT APPLICATION

(11) **EP 4 400 131 A1**
(43) Date of publication of application: **17.07.2024**
(21) Application number: 23151065.2
(22) Date of filing: 11.01.2023
(51) Int. Cl.: A61M 5/24, A61M 5/28, A61M 5/31, A61M 5/315

(54) **CARTRIDGE SYRINGE**

(71) Applicant: Activoris Medizintechnik GmbH, 35285 Gemünden (Wohra) (DE)
(72) Inventor: FISCHER, Axel, 34630 Gilserberg (DE); DUNNE, Stephen T., Porto 4150-044 (PT)
(74) Representative: Tergau & Walkenhorst Intellectual Property GmbH

(57) **Abstract**

A cartridge syringe comprising an outer casing (2) for receiving a cartridge (3), and a displaceably guided plunger (4) disposed at the rear end of the outer casing (2), wherein the plunger (4) comprises an outer sleeve (43) having a guiding channel (49), and a core (41) disposed in the guiding channel (49) of the outer sleeve (43), so that the core (41) can be moved along its longitudinal axis in the guiding channel (49) of the outer sleeve (43) and relative to the outer sleeve (43), wherein the cartridge syringe comprises at least one spiral guiding groove (25) arranged at the inner surface of the outer casing (2), or arranged at the outer surface of the outer sleeve (43), so that upon rotating the outer sleeve (43) of the plunger (4) in the outer casing (2), the outer sleeve (43) is forcing the distal end of the plunger (48) to engage with the proximal end (33) of the cartridge (3) in a radial rotational movement.

## Description

The present invention relates to a cartridge syringe, a cartridge for such cartridge syringe, a method for applying a drug, a medicament or other formulation to a subject with such cartridge syringe or cartridge, respectively, and the use of such cartridge syringe.

Cartridge syringes are known in the art. A cartridge syringe is a special type of syringe. It is particularly used in dentistry, usually for local anaesthesia, e.g. before tooth extraction. The cartridge syringe is equipped by inserting a cylinder cartridge comprising a cylindrical ampule, usually in the form of a glass ampule cylinder, that is already filled sterile with a drug or other formulation at the pharmaceutical manufacturer. Usually, the ampule cylinder filled with the drug is closed at the front end by means of a needle pierceable septum and at the rear end by means of a stopper. The needle pierceable septum is pierced by an injection needle for injection in the manner of an injection vial. The stopper at the rear end is then pressed into the ampule cylinder like a piston by the pressure of the punch of the cartridge syringe and thus the sterile drug is injected via the cannula into the desired injection site. Usually, special cannulas, so-called cartridge cannulas, are required for the cartridge syringe. They resemble a normal cannula of the ground hollow tube on the long side, with which the local anaesthetic is injected, and have another short ground hollow tube at the rear end, with which the puncture diaphragm of the cylinder ampoule is pierced. The cartridge cannulas are screwed onto the cartridge syringes.

The main advantage of using a cartridge syringe compared to a standard injection cannula is that the drug does not have to be pulled out of an ampoule, and instead is easily loaded by inserting the cartridge into the syringe. Also, a practitioner may reload a second cartridge single handed, and the risk of losing sterility is reduced. Thereby, the longitudinal design of the cartridge syringe, due to the narrow cylinder ampoules, also allows the dentist a higher visibility of drug and application during injection. With a short syringe, the patient's mouth is more difficult to access and disadvantageously hinders also illumination and the field of view.

However, there exists also a disadvantage in the use of cartridge syringes in that the handling of the cartridge, particularly its proper insertion and the engagement of the plunger with the stopper of the cartridge can be bothersome and error prone. Depending on the model, the cartridge is either inserted into the cartridge syringe from the back after the plunger has been removed, or the cartridge is inserted into the syringe from the side. Standard cylinder ampoule cartridges have a volume of about 1.7 ml. Compared to disposable syringes, this requires a relatively long syringe piston, and a relatively long plunger. As a result, the cartridge syringes as such are relatively long. While the length allows a more precise dosing and a slower injection upon use, the proper insertion of the cartridge, single-handed operations (i.e. aspiration movements) are challenging in case of smaller hands (e.g. women). Further, the engagement of the plunger with the stopper of the cartridge, as well as a necessary aspiration movement causes problems, if the cartridge is canted or inserted at an angle into the syringe.

The document US 2014/0299228 A1 describes a cartridge syringe, where a front end of the outer sleeve is provided with a fixing hook for the attachment in the blind hole of the stopper of the cartridge. This allows an engagement of the plunger and the cartridge stopper. However, the described solution is relatively complex, the fixing hooks made of plastics may abort. Also, this document is silent about how to prevent the cartridge being canted or inserted at an angle into the syringe.

It is an object of the present invention to provide a cartridge syringe that has improved aspiration properties. It is a further object of the present invention to provide a cartridge syringe where the loading and engagement of the cartridge is improved and particularly a cartridge is prevented from canting or insertion at an angle into the syringe.

These and other problems are solved by the subject matter of the attached independent claims.

The above objects of the invention are achieved by a cartridge syringe, a cartridge, a method for applying a drug, a medicament or other formulation to a subject and a use of the cartridge syringe according to the annexed independent claims.

Preferred embodiments may be taken from the dependent claims, and, beyond that, from the following description, in particular comprising various embodiments as covered and described in the annexed claims.

A cartridge syringe according to the present invention comprises an outer casing for receiving a cartridge, and a displaceably guided plunger disposed at the rear end of the outer casing, wherein the plunger comprises an outer sleeve having a guiding channel, and a core disposed in the guiding channel of the outer sleeve. In such configuration the core can be moved along its longitudinal axis in the guiding channel of the outer sleeve and relative to the outer sleeve. Such cartridge syringe according to the present invention comprises at least one spiral guiding groove arranged at the inner surface of the outer casing, or arranged at the outer surface of the outer sleeve.

In a preferred embodiment and in accordance with an aspect of the present invention, this guiding groove positioned either at the inner surface of the outer casing or at the outer surface of the outer sleeve interacts with an associated, corresponding engagement member, in particular a cam, positioned at the outer surface of the outer sleeve or the inner surface of the outer casing, respectively. The pairing of these components, i. e. the cam-type engagement member engaging the corresponding guiding grooves, provides means for converting a rotational motion of the plunger relative to the outer casing into a translational displacement of the plunger relative to and within the outer casing, due to the spiral, thread-like configuration of the guiding groove. Therefore, superior control regarding the displacement of the plunger can be provided for the user, since the rotation of the components relative to each other may be effected with high precision.

In a preferred embodiment and according to one aspect of the invention, the components mentioned above, in particular the cams and the spiral groove, with respect to their characteristic parameters such as thread lift etc are provided in such configuration that upon rotating the outer sleeve of the plunger in the outer casing, the outer sleeve is forcing the distal end of the plunger to engage with the proximal end of the cartridge in a radial rotational movement.

This is of particular advantage, as the rotational movement of the outer sleeve causes a defined feed motion of the plunger and the distal end of the plunger is forced to engage with the proximal end of the cartridge. This allows a more secure loading and engagement of the cartridge and particularly prevents the cartridge from being canted or inserted in an angle. Additionally, the proper engagement of the cartridge by the plunger facilitates the aspiration.

Within the present application, terms such as "lateral" or "laterally", "rear", "frontal", "upper", "lower", "bottom", "opposite", "inner", "outer", "proximal", "distal" or the like, as used herein, which de-scribe the position of a first object relative to another object, preferably refer to the relative position of a respective part or object regarding its position fully mounted for its intended use. Particularly, the terms such as "proximal" or "distal" thereby preferably refer to a position relative to the medical practitioner holding the inventive cartridge syringe in its hands for the intended use of injecting a drug, medicament or other formular to a subject. In an advantageous embodiment of the inventive method said cartridge syringe comprises at least one engagement member arranged to engage with and to be guided in the at least one spiral guiding groove.

Such engagement member advantageously provides a more secured guiding of the outer sleeve along the spiral guiding groove and thus facilitates the advantageous feed motion of the plunger for engagement of the cartridge.

In a further advantageous embodiment of the inventive cartridge syringe, when the spiral guiding groove is arranged at the inner surface of the outer casing, the at least one engagement member is arranged at the outer surface of the outer sleeve. Such embodiment is particularly preferred and improves the insertion of the plunger into the outer sleeve and subsequently facilitates the advantageous feed motion of the plunger for engagement of the cartridge. Additionally, or alternatively, when the spiral guiding groove is arranged at the outer surface of the outer sleeve, the at least one engagement member is arranged at the inner surface of the outer casing.

In a further advantageous embodiment of the inventive cartridge syringe, the cartridge syringe comprises a pair of two engagement members, and a pair of two spiral guiding grooves, arranged such that the first engagement member engages with the first spiral guiding groove and the second engagement member engages with the second spiral guiding groove. A plurality of pairs of engagement member and respective guiding groove thereby improves the insertion of the plunger into the outer sleeve and subsequently facilitates the advantageous feed motion of the plunger for engagement of the cartridge. Additionally, the arrangement can be provided in a poka-yoke configuration, so that the plunger can be inserted into the outer sleeve in only one desired configuration.

In a further advantageous embodiment of the inventive cartridge syringe, the engagement member is provided in the form of a pencil-shaped protrusion or a pin. Such pencil-shaped protrusion or pin provides an advantageously simple engagement and guiding of the engagement member with and in the spiral guiding groove.

In a further advantageous embodiment of the inventive cartridge syringe, one pair of engagement members is arranged at the outer surface of the outer sleeve and one pair of one pair of spiral guiding grooves is arranged at the inner surface of the outer casing, wherein preferably the engagement members are arranged at opposite sides of the outer surface of the outer sleeve, and the spiral guiding grooves are arranged at the opposite sides of the inner surface of the outer casing. Such arrangement was found by the present inventors to be preferred as only two pairs of engagement members and spiral guiding grooves are needed and thus cost and material effective. The arrangement at opposite sides at the outer surface of the outer sleeve and the inner surface of the outer casing, respectively, thereby secures a proper fit and facilitates guiding of the outer sleeve in the outer casing. Of course, additionally or alternatively, the respective reverse arrangement is also considered herein, wherein one pair of engagement members is arranged at the inner surface of the outer casing and one pair of spiral guiding grooves is arranged at the outer surface of the outer sleeve, wherein preferably the engagement members are arranged at opposite sides of the inner surface of the outer casing, and the spiral guiding grooves are arranged at the opposite sides of the outer surface of the outer sleeve.

In a further advantageous embodiment of the inventive cartridge syringe, the upper rim section located at the rear end of the outer sleeve comprises an actuation means. Such actuation means allows the user to engage with the outer sleeve more easily and facilitates thus the insertion of the plunger, particularly of the outer sleeve, into the outer casing, and subsequently the rotational movement of the outer sleeve that causes a defined feed motion of the plunger, whereby the distal end of the plunger is forced to engage with the proximal end of the cartridge. Such actuation means can be provided in different ways. For example, and as preferred herein, the actuation means is formed as a circumferential protrusion, which facilitates its actuation. More preferably such actuation means, and particularly such circumferential protrusion may comprise a lateral rim fluting, that provides a particular secure hold, and prevents slipping of the fingers. Particularly, with such configuration a one-handed handling of the cartridge syringe is facilitated.

In a further advantageous embodiment of the inventive cartridge syringe, the spiral guiding groove comprises at the rear end of the outer casing or the frontal end of the outer sleeve, respectively, a straight introduction part arranged parallel to the longitudinal axis of the cartridge syringe. The present inventors have found that such, preferably relatively short, straight channel part facilitates the insertion of the outer sleeve into the outer casing. After said straight introduction part the spiral guiding groove can be more easily engaged.

In a further advantageous embodiment of the inventive cartridge syringe, the spiral guiding groove comprises at least one complete coil winding in relation to the longitudinal axis of the cartridge syringe. Providing at least one complete coil winding is advantageous to cause the rotational movement of the outer sleeve a defined feed motion of the plunger, so that the distal end of the plunger is more properly forced to engage with the proximal end of the cartridge.

In a further advantageous embodiment of the inventive cartridge syringe, the core of the plunger comprises at its distal end an engagement means configured to engage with the proximal end of the cartridge, preferably to engage with a stopper provided at the proximal end of the cartridge. Such engagement means may be provided preferably in a plug shape. The engagement means is forced by the radial rotational movement to connect with the cartridge, particularly the stopper, preferably providing a respective backforming, which allows a proper connection of the cartridge and the plunger, and thus facilitates a respective aspiration movement.

In a further advantageous embodiment of the inventive cartridge syringe, the core of the plunger comprises at its distal end a core stopper. Such core stopper can advantageously secure that the plunger is engaging with the cartridge and is prevented from slipping out of the proximal end of the guiding channel of the outer sleeve. Such core stopper may, for example, be provided in the form of a protrusion, or particularly circumferential protrusion of the core of the plunger.

In a further advantageous embodiment of the inventive cartridge syringe, the outer sleeve is provided as a bipartite outer sleeve, comprising a first outer sleeve part and a second outer sleeve part, and wherein preferably each outer sleeve part is identical to each other. This advantageously facilitates the assembly process of the cartridge syringe and particularly of providing the outer sleeve to the plunger around the core of the plunger.

In a further advantageous embodiment of the inventive cartridge syringe, each of the first outer sleeve part and the second outer sleeve part comprises at least one connecting hole and at least one connecting plug, wherein each connecting plug is configured to engage with the connecting hole of the respective other sleeve part. This allows that the first outer sleeve part and the second outer sleeve part can be plugged together by engaging each connecting plug, with a respective connecting hole to form a bipartite outer sleeve. Also this means provides an easy disassembly of the outer sleeves and the ram for sterilization.

The above-described problems are also advantageously solved by a cartridge according to the present invention. Such cartridge is for use in a cartridge syringe according to the present invention, and comprises an ampule cylinder, a cap assembly, and a stopper. The cap assembly comprises a needle pierceable septum or sealing element held by a ferrule or crimp at the distal end of the ampule cylinder. The ampule cylinder is provided to contain a drug or medicament or other formulation between the stopper and the septum or sealing element of the cap assembly. The stopper is disposed at the proximal end of the ampule cylinder. Advantageously, the stopper is manufactured from an elastic material, preferably a plastic or resin, and comprises a blind hole for engagement by an engagement means of a core of a plunger of the cartridge syringe. Such blind hole preferably is disposed in the form of a backforming provided to engage with engagement means of plunger. This facilitates proper engagement of the plunger, particularly the distal end of the core, and the stopper of the cartridge and facilitates aspiration movement.

In an alternative, independently inventive embodiment, engagement of the plunger with the stopper may be achieved by appropriate design of the distal end of the plunger. In an embodiment, the plunger rod might be designed to provide 'engagement means' for example in the form of a 'Harpoon'-type design forming barbs or similar forms. In this embodiment the stopper, provided it is made from sufficiently soft or flexible material, does not need a blind hole but may be pierced by the distal end of the plunger rod directly. In particular, relatively large diameter stoppers typically do not have a hole, so for these this embodiment is considered an advantage.

The above-described problems are also advantageously solved by a method according to the present invention. A method for applying a drug, a medicament or other formulation to a subject according to the present invention, comprises at least the following steps:
- a step a) of inserting a cartridge, preferably according to the present invention, into the outer casing of a cartridge syringe according to the present invention;
- a step b) of engaging a displaceably guided plunger with the outer casing of the cartridge syringe by engagement of at least one engagement member with a respective spiral guiding groove, wherein
   o when the spiral guiding groove is arranged at the inner surface of the outer casing, the at least one engagement member is arranged at the outer surface of the outer sleeve
   o and/or
   o wherein when the spiral guiding groove is arranged at the outer surface of the outer sleeve, the at least one engagement member is arranged at the inner surface of the outer casing;
- a step c) of inserting the outer sleeve into the outer casing by actuating an actuation means, preferably formed as a circumferential protrusion, more preferably comprising a lateral rim fluting, at the rear end of the outer sleeve so that upon rotating the outer sleeve of the plunger in the outer casing, the outer sleeve is forcing the distal end of the plunger to engage with the stopper at the proximal end of the cartridge in a radial rotational movement, and
- a step d) of actuating the actuating element of the core of the plunger, so that the core is moved along its longitudinal axis in the guiding channel of the outer sleeve and relative to the outer sleeve and thereby the engaged stopper at the proximal end of the cartridge is pushed into the ampule cylinder.

A skilled person will immediately understand that in addition to a step d) wherein the engaged stopper at the proximal end of the cartridge is pushed into the ampule cylinder, which forces the injection of the drug, medicament or other formulation, a step of aspiration movement may be comprised in the inventive method. Thereby, the actuating of the actuating element of the core of the plunger, so that the core is moved along its longitudinal axis in the guiding channel of the outer sleeve and relative to the outer sleeve is to pull the engaged stopper slightly from the proximal end of the cartridge and thus cause a vacuum in the ampule cylinder.

It shall be noted, that the steps given above do not necessarily have to be carried out in the given, although preferred, order. The provided steps may be carried out in any other suitable order as far as meaningful for the intended use.

However, the order as given above may apply for particular variants of the method. For example, an aspiration step may be performed prior to a step d) of injection.

It will be immediately acknowledged by a person skilled in the art that a feature, embodiment, effect or advantage described herein in connection with the inventive cartridge or cartridge syringe, may also be a feature, embodiment, effect or advantage of the inventive method, respectively, and vice versa.

The above described problems are also advantageously solved by a use of the inventive cartridge syringe according to the present invention. Such use of the cartridge syringe according to the present invention is for applying a drug, a medicament or another formulation to a subject.

The present invention will be described in further detail with reference to the drawings from which further features, embodiments and advantages may be taken, and in which:
- FIG 1A: illustrates a perspective view of a first embodiment of the cartridge syringe loaded with a cartridge;
- FIG 1B: illustrates a perspective exploded view of the first embodiment of the cartridge syringe;
- FIG 2A: illustrates a perspective exploded view of the outer casing of the first embodiment of the cartridge syringe;
- FIG 2B: illustrates a side view of the outer casing of the first embodiment of the cartridge syringe;
- FIG 2C: illustrates a perspective view of the rear end of the outer casing of the first embodiment of the cartridge syringe;;
- FIG 3A and 3B: illustrate perspective views of the plunger of the first embodiment of the cartridge syringe;
- FIG 4: illustrates a perspective view of the core of the plunger of the first embodiment of the cartridge syringe;
- FIG 5A: illustrates a perspective view of the first outer sleeve part of the plunger of the first embodiment of the cartridge syringe;
- FIG 5B: illustrates a side view of the first outer sleeve part of the plunger of the first embodiment of the cartridge syringe;
- FIG 6A and 6B: illustrates perspective views of the second embodiment representing cartridge of the present invention.

In the embodiments shown in the figures, elements similar or identic in function are designated with like reference signs. It is noted, that the figures may not be true to scale with respect to each other.

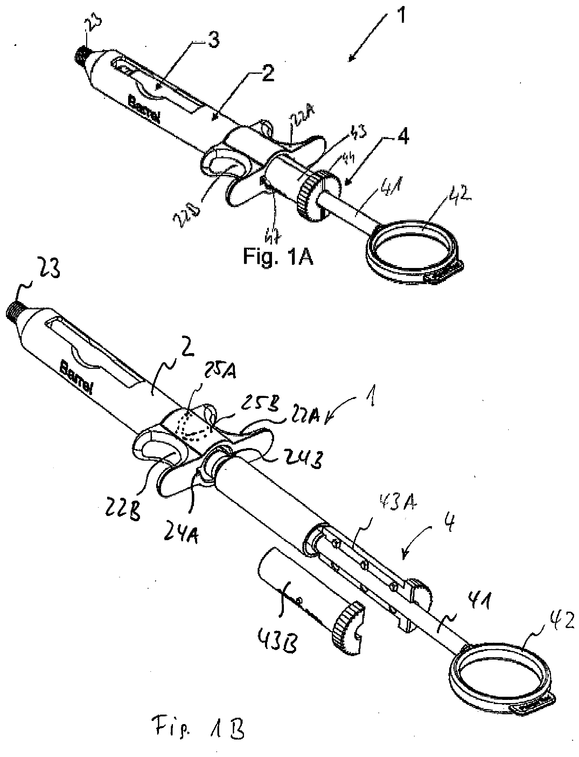

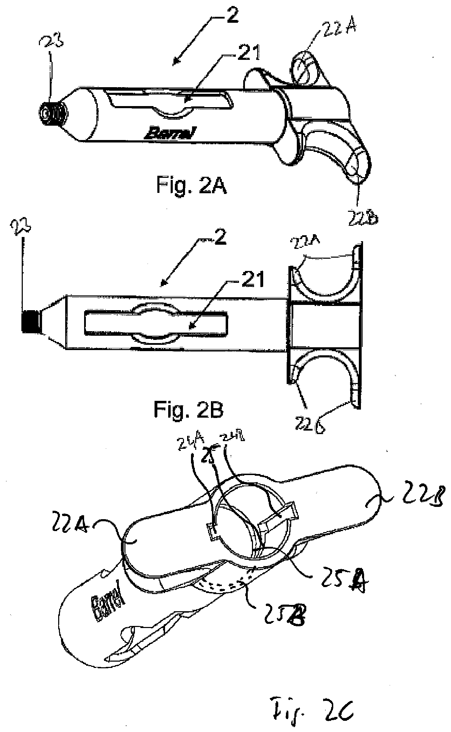

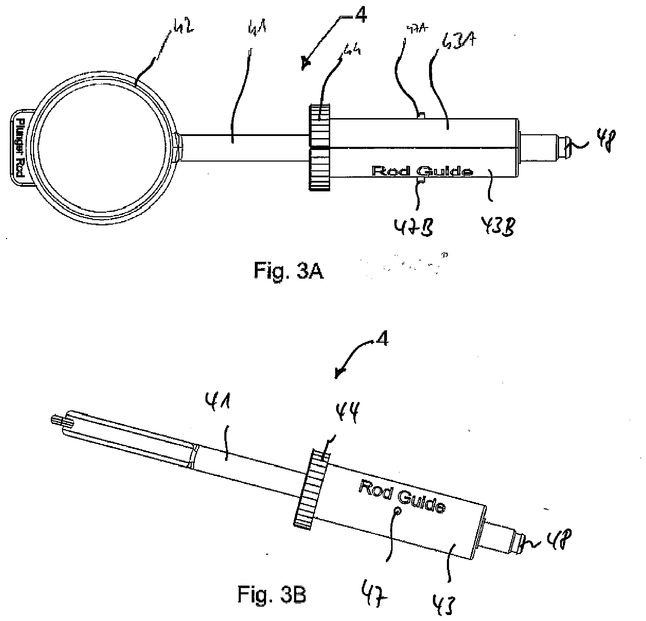

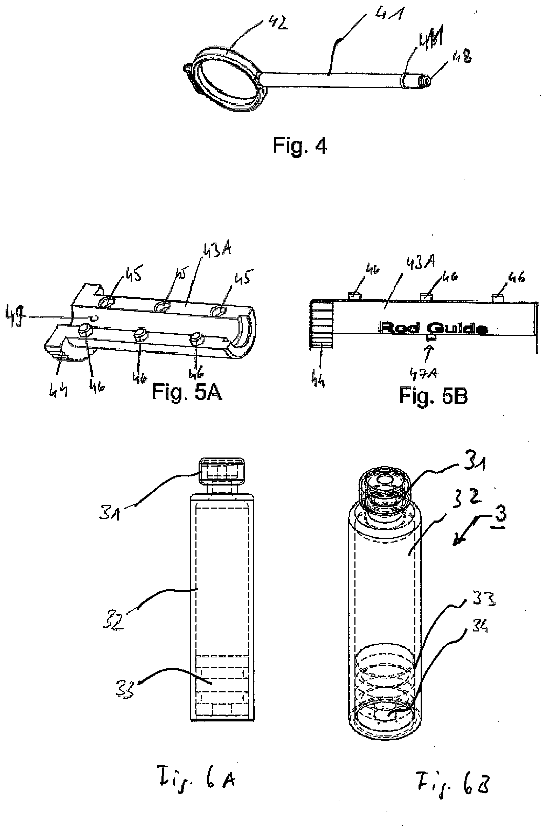

FIG 1 shows a perspective view of a first embodiment of the cartridge syringe 1 loaded with a cartridge 3. The cartridge syringe 1 according to the first embodiment of the present invention comprises an outer casing 2 for receiving a cartridge 3, and a displaceably guided plunger 4 disposed at the rear end of the outer casing 2. In FIG 1B a perspective exploded view of the first embodiment of the cartridge syringe is shown. With the cartridge syringe 1 according to the first embodiment, particularly the method according to the present invention can be carried out. Preferably, prior to or after the steps of the inventive method a respective syringe needle (not shown) may be assembled to the cartridge syringe 1 of the present invention. For this purpose a syringe injection needle is attached, preferably screwed, to the connector 23 at provided at the distal end of the cartridge syringe 1 capable of holding said needle arrangement. Particularly, such needle arrangement may comprise a septum needle 124 and an injection needle where the connector 23 is a threaded connector as shown here, or alternatively a coiled tip, a luer connector or any other suitable connector. In a step a) of the method according to the present invention the cartridge 3 is inserted into the outer casing 2 of the cartridge syringe. For this purpose, either the plunger 4 is removed from the outer casing 2 and the cartridge 3 is inserted into the proximal opening of the outer casing 2, or, alternatively the outer casing 2 comprises lateral longitudinal openings 21, as shown in FIG 2A or FIG 2B that are dimensions such that the cartridge 3 can be inserted into the outer casing 2 from the side and through the lateral longitudinal openings 21. Subsequently, in a step b) the displaceably guided plunger 4 is engaged with the outer casing 2 of the cartridge syringe 1. This engagement is facilitated by at least one engagement member 47, best seen in FIG 3A or 3B, with a respective spiral guiding groove 25. In a further subsequent step c) the outer sleeve 43 is inserted into the outer casing 2 by actuating the actuation means 44, here formed as a circumferential protrusion with a lateral rim fluting, at the rear end of the outer sleeve 43. Thereby, upon rotating the outer sleeve 43 of the plunger 4 in the outer casing 2, the outer sleeve 43 is forcing the distal end of the plunger 48 to engage with the stopper 33 at the proximal end of the cartridge 3 in a radial rotational movement. This radial rotational movement of the outer sleeve 43 causes a defined feed motion of the plunger 4 and the distal end of the plunger 48 is engaging with the proximal end 33 of the cartridge 3 thereby securing loading and engagement of the cartridge 3 and particularly preventing the cartridge 3 from being canted or inserted in an angle in the outer casing 2. As immediately visible from FIG 2B, the proper engagement of the cartridge 3 by the plunger 4 also facilitates the aspiration upon pulling the actuating element 42. Such actuating element may be in the form of a finger pad or - as shown here - a finger ring 42. In such a step of aspiration movement the actuating of the actuating element 42 of the core 41 of the plunger 4 the core 41 is moved along its longitudinal axis in the guiding channel 49 of the outer sleeve 43. Thereby relative to the outer sleeve 43 the engaged stopper 33 is slightly pulled from the proximal end 33 of the cartridge 3 and thus a vacuum is caused in the ampule cylinder 32 resulting in an aspiration. Subsequent to aspiration, the injection of the medicament or drug can be carried out in a step d) by actuating the actuating element 42 of the core 41 of the plunger 4 in the other direction, particularly by moving the core 41 along its longitudinal axis in the guiding channel 49 of the outer sleeve 43 and relative to the outer sleeve 43 and thus pushing the engaged stopper 33 at the proximal end of the cartridge 3 into the ampule cylinder 32. As particularly visible in FIGs 3A and 3B the plunger 4 comprises an outer sleeve 43 comprising a guiding channel 49 in a central hollow along its longitudinal axis as visible from FIG 5A in particular. In addition the plunger 4 has a core 41 that is shown in detail in FIG 4, which is disposed in the guiding channel 49 of the outer sleeve 43 of the plunger 4.

As obvious from FIG 1B, FIG 2C and FIG 3A in particular, in the shown embodiment the cartridge syringe 1 comprises a pair of two engagement members 47A and 47B, and a pair of two spiral guiding grooves 25A and 25B, arranged such that the first engagement member 47A engages with the first spiral guiding groove 25A and the second engagement member 47B engages with the second spiral guiding groove 25B, which improves the insertion of the plunger 4 into the outer sleeve 43 and subsequently facilitates the advantageous feed motion of the plunger 4 for engagement of the cartridge 3.

Particularly, one pair of engagement members 47A and 47B is arranged at the outer surface of the outer sleeve 43 and one pair of one pair of spiral guiding grooves 25A and 25B is arranged at the inner surface of the outer casing 2, with the engagement members 47A and 47B being arranged at opposite sides of the outer surface of the outer sleeve 43, and the spiral guiding grooves 25A and 25B being arranged at the opposite sides of the inner surface of the outer casing 2. Thereby, the provision of only two pairs of engagement members 47A and 47B and spiral guiding grooves 25A and 25B, respectively is advantageously a cost and material effective way to facilitate proper engagement of the plunger 4 in the outer housing 2. The arrangement at opposite sides at the outer surface of the outer sleeve 43 and the inner surface of the outer casing 2, respectively, thereby secures a proper fit and facilitates guiding of the outer sleeve 43 in the outer casing 2.

FIGs 2A, 2B and 2C show different detailed views of the outer casing 2 of the first embodiment of the cartridge syringe 1. Such outer casing is provided in the form of an overall cylindrical shape, having a distal connector 23 for connecting an injection cannula, a central portion with a longitudinal hollow for receiving the cartridge 3, and proximal holding elements 22A and 22B in the form of finger flanges, or alternatively finger pads or finger rings that facilitate holding of the cartridge syringe 1 by the user, particularly in one-handed operation.

Particularly from FIG 2C it can be taken that the spiral guiding groove 25 is arranged at the inner surface of the outer casing 2. In this configuration the insertion of the plunger 4 into the outer sleeve 43 is improved and particularly the advantageous feed motion of the plunger 4 for engagement of the cartridge 3 is facilitated. As visible particularly from FIG 2C the spiral guiding groove 25, 25A, 25B comprises at least one complete coil winding in relation to the longitudinal axis of the cartridge syringe 1 so that the rotational movement of the outer sleeve 43 defines the feed motion of the plunger 4 optimally to force the distal end of the plunger to engage properly with the proximal end of the cartridge 3. Furthermore, the spiral guiding groove 25, 25A, 25B comprises at the rear end of the outer casing 2 a straight introduction part arranged parallel to the longitudinal axis of the cartridge syringe 1 that facilitates the insertion of the outer sleeve 43 into the outer casing 2. After said straight introduction part the spiral guiding groove 25, 25A, 25B can be more easily engaged. In such configuration and upon rotating the outer sleeve 43 of the plunger 4 in the outer casing 2, the outer sleeve 43 is forcing the distal end of the plunger 48 to engage with the proximal end 33 of the cartridge 3 in a radial rotational movement.

Particularly, FIG 4 shows a perspective view of the core 41 of the plunger 4 of the first embodiment of the cartridge syringe 1, wherein the core 41 comprising a core stopper 411 at its distal end 48 in the form of a circumferential protrusion of the core 41 that secures that the plunger 4 is engaging with the cartridge 3 and is prevented from slipping out of the proximal end of the guiding channel 49 of the outer sleeve 43.

In addition the core 41 comprises at its distal end 48 a plug-shaped engagement means 48 configured to engage with the proximal end of the cartridge 3, particularly with the stopper 33 provided at the proximal end of the cartridge 3. The plug 48 is forced by the radial rotational movement to connect with the cartridge 3, particularly the stopper 33, particularly with the blind hole 34 of the stopper 33 providing a respective backforming, which allows a proper connection of the stopper 33 and the plug 48, which allows a respective aspiration movement of the plunger backwards towards the proximal end of the outer housing 2.

FIGs 5A and and FIG 5B show the first outer sleeve part 43A of the plunger 4 of the first embodiment of the cartridge syringe 1. A person skilled in the art will immediately acknowledge that each of the first outer sleeve part 43A and the identical second outer sleeve part 43B comprises three connecting holes 45 and three respective connecting plugs 46. Each connecting plug 46 is configured to engage with one respective connecting hole 45 of the respective other sleeve part 43A, 43B, respectively. This allows that the first outer sleeve part 43A and the second outer sleeve part 43B can be plugged together by engaging each connecting plug 46, with a respective connecting hole 45 to form a bipartite outer sleeve 43 comprising the central guiding channel 49.

It is immediately apparent that the core 41 thus can be moved along its longitudinal axis in the guiding channel 49 of the outer sleeve 43 and relative to the outer sleeve 43. Such cartridge syringe according to the present invention comprises at least one spiral guiding groove 25 arranged at the inner surface of the outer casing 2, or arranged at the outer surface of the outer sleeve 43.

Also visible is that the engagement member 47, 47A of the first outer sleeve part 43A, and thus the engagement member 47, 47B of the identical second outer sleeve part 43B, is provided in the form of a pin. Such pin 47A, 47B provides an advantageously simple engagement and guiding of the pins 47A, 47B with and in the spiral guiding grooves 25A, and 25B, respectively.

FIGs 3A and 3B show perspective views of the plunger 4 of the first embodiment of the cartridge syringe 1. It can be taken therefrom that the engagement members 47A and 47B are arranged at the outer surface of the outer sleeve 43A and 43B, respectively. Thereby each engagement member 47, 47A, 47B is arranged to engage with and to be guided in the at least one spiral guiding groove 25, 25A, 25B, respectively, and thus a more secured guiding of the outer sleeve 43 along the spiral guiding grooves 25A, 25B is provided, facilitating the advantageous feed motion of the plunger 4 to securely engage the cartridge 3 in a controlled manner.

Additionally, the upper rim section located at the rear end of the outer sleeve 43 comprises an actuation means 44 formed as a circumferential protrusion with a lateral rim fluting 44 that allows the user to engage with the outer sleeve 43 more easily and facilitates thus the insertion of the plunger 4, particularly of the outer sleeve 43, into the outer casing 2, and subsequently the rotational movement of the outer sleeve 43 that causes a defined feed motion of the plunger 4, whereby the distal end of the plunger 4 is forced to engage with the proximal end of the cartridge 3.

Such cartridge is exemplified in FIG 6A and 6B that show perspective views of the second embodiment representing a cartridge 3 of the present invention. The cartridge 3 is particularly useful in a cartridge syringe 1 according to the first embodiment and comprises an ampule cylinder 32, a cap assembly 31, and a stopper 33. The ampule cylinder 32 is provided in the form of a generally tubular glass or plastic barrel to comprise the medicament, drug or other formulation for the intended use. The cap assembly 31 comprises a needle pierceable septum or sealing element held by a ferrule or crimp at the distal end of the ampule cylinder 32. The ampule cylinder 32 is provided to contain a drug or medicament or other formulation between the stopper 33 and the septum or sealing element of the cap assembly 31. The stopper 33 is disposed at the proximal end of the ampule cylinder 32 and manufactured from an elastic plastic material. The stopper 33 in the form of a rubber piston 33, is disposed at the proximal end of the ampule cylinder 32 to contain the medicament or other formulation between the stopper/piston member 33 and the septum or sealing element at the distal end. The stopper 33 comprises a blind hole 34 that represents a backforming, which allows a proper connection and engagement of the stopper 33 and the plug 48 of the core 41 of the plunger 4. Such connection is detachable so that the cartridge 3 can be thrown away after use and the cartridge syringe 1 may be reused with another, fresh cartridge 3. However, the connection by clamping the stopper 33 into the elastic blind hole 34 is tight enough to advantageously allow a backwards aspiration movement of the plunger 4.

The embodiments in the figures may relate to preferred embodiments, while all elements and features described in connection with embodiments may be used, as far as appropriate, in combination with any other embodiment and feature as discussed herein, in particular related to any other embodiment discussed further above.

### List of reference numerals

- 1: Cartridge Syringe
- 2: outer casing
- 21: lateral longitudinal opening
- 22A, 22B: holding element
- 23: connector
- 25, 25A, 25B: spiral guiding groove
- 3: cartridge
- 31: cap assembly
- 32: ampule cylinder
- 33: stopper
- 34: blind hole of the stopper
- 4: displaceably guided plunger
- 41: core
- 411: core stopper
- 42: actuating element
- 43, 43A, 43B: outer sleeve
- 44: actuating means
- 45: connecting hole
- 46: connecting plug
- 47, 47A, 47B: engagement member
- 48: engagement means
- 49: guiding channel

## Claims

1. A cartridge syringe (1) comprising
an outer casing (2) for receiving a cartridge (3), and a displaceably guided plunger (4) disposed at the rear end of the outer casing (2),
wherein the plunger (4) comprises an outer sleeve (43) having a guiding channel (49), and a core (41) disposed in the guiding channel (49) of the outer sleeve (43)
wherein
the cartridge syringe (1) comprises at least one spiral guiding groove (25) arranged at the inner surface of the outer casing (2) or arranged at the outer surface of the outer sleeve (43).

2. The cartridge syringe (1) according to claim 1, comprising at least one engagement member (47) arranged to engage with and to be guided in the at least one spiral guiding groove (25).

3. The cartridge syringe (1) according to claim 2, wherein
when the spiral guiding groove (25) is arranged at the inner surface of the outer casing (2), the at least one engagement member (47) is arranged at the outer surface of the outer sleeve (43)
and/or
wherein when the spiral guiding groove (25) is arranged at the outer surface of the outer sleeve (43), the at least one engagement member (47) is arranged at the inner surface of the outer casing (2).

4. The cartridge syringe (1) according to any one of claims 1 to 3, comprising a pair of two engagement members (47A and 47B), and a pair of two spiral guiding grooves (25A and 25B), arranged such that the first engagement member (47A) engages with the first spiral guiding groove (25A) and the second engagement member (47B) engages with the second spiral guiding groove (25B).

5. The cartridge syringe (1) according to any one of claims 2 to 4, wherein the engagement member (47) is provided in the form of a pencil-shaped protrusion or a pin.

6. The cartridge syringe (1) according to any of claims 1 to 5, wherein one pair of engagement members (47A and 47B) is arranged at the outer surface of the outer sleeve (43) and one pair of one pair of spiral guiding grooves (25A and 25B) is arranged at the inner surface of the outer casing (2), wherein preferably the engagement members (47A and 47B) are arranged at opposite sides of the outer surface of the outer sleeve (43), and the spiral guiding grooves (25A and 25B) are arranged at the opposite sides of the inner surface of the outer casing (2)
and/or
wherein one pair of engagement members (47A and 47B) is arranged at the inner surface of the outer casing (2) and one pair of one pair of spiral guiding grooves (25A and 25B) is arranged at the outer surface of the outer sleeve (43), wherein preferably the engagement members (47A and 47B) are arranged at opposite sides of the ) inner surface of the outer casing (2), and the spiral guiding grooves (25A and 25B) are arranged at the opposite sides of the outer surface of the outer sleeve (43).

7. The cartridge syringe (1) according to any of claims 1 to 6, wherein the upper rim section located at the rear end of the outer sleeve (43) comprises an actuation means, preferably formed as a circumferential protrusion, more preferably comprising a lateral rim fluting (44).

8. The cartridge syringe (1) according to any of claims 1 to 7, wherein the spiral guiding groove (25, 25A, 25B) comprises at the rear end of the outer casing (2) or the frontal end of the outer sleeve (43, 43A, 43B), respectively, a straight introduction part arranged parallel to the longitudinal axis of the cartridge syringe (1).

9. The cartridge syringe (1) according to any of claims 1 to 8, wherein the spiral guiding groove (25, 25A, 25B) comprises at least one complete coil winding in relation to the longitudinal axis of the cartridge syringe (1).

10. The cartridge syringe (1) according to any of claims 1 to 9, wherein the core (41) of the plunger (4) comprises at its distal end (48) an engagement means (48) configured to engage with the proximal end of the cartridge (3), preferably to engage with a stopper (33) provided at the proximal end of the cartridge (3).

11. The cartridge syringe (1) according to any of claims 1 to 10, wherein the outer sleeve (43) is provided as a bipartite outer sleeve (43), comprising a first outer sleeve part (43A) and a second outer sleeve part (43B), and wherein preferably each outer sleeve part (43A, 43B) is identical to each other.

12. The cartridge syringe (1) according to any of claims 1 to 11, wherein each of the first outer sleeve part (43A) and the second outer sleeve part (43B) comprises at least one connecting hole (45) and at least one connecting plug (46), wherein each connecting plug (46) is configured to engage with the connecting hole (45) of the respective other sleeve part.

13. A cartridge (3), particularly a cartridge for use in a cartridge syringe (1) according to any one of claims 1 to 12, comprising an ampule cylinder (32), a cap assembly (31), and a stopper (33), the cap assembly (31) comprising a needle pierceable septum or sealing element held by a ferrule or crimp at the distal end of the ampule cylinder (32), the ampule cylinder (32) provided to contain a drug or medicament or other formulation between the stopper (33) and the septum or sealing element of the cap assembly (31), the stopper (33) disposed at the proximal end of the ampule cylinder (32), wherein the stopper (33) manufactured from an elastic material, preferably a plastic or resin, and comprising a blind hole (34) for engagement by an engagement means (48) of a core (41) of a plunger (4) of a cartridge syringe (1).

14. A method for applying a drug, a medicament or other formulation to a subject, comprising at least the following steps comprising
- a step a) of inserting a cartridge (3), preferably according to claim 13, into the outer casing (2) of a cartridge syringe (1) according to any one of claims 1 to 12;
- a step b) of engaging a displaceably guided plunger (4) with the outer casing (2) of the cartridge syringe (1) by engagement of at least one engagement member (47) with a respective spiral guiding groove (25), wherein
o when the spiral guiding groove (25) is arranged at the inner surface of the outer casing (2), the at least one engagement member (47) is arranged at the outer surface of the outer sleeve (43)
o and/or
o wherein when the spiral guiding groove (25) is arranged at the outer surface of the outer sleeve (43), the at least one engagement member (47) is arranged at the inner surface of the outer casing (2);
- a step c) of inserting the outer sleeve (43) into the outer casing (2) by actuating an actuation means (44), preferably formed as a circumferential protrusion, more preferably comprising a lateral rim fluting (44), at the rear end of the outer sleeve (43) so that upon rotating the outer sleeve (43) of the plunger (4) in the outer casing (2), the outer sleeve (43) is forcing the distal end of the plunger (48) to engage with the stopper (33) at the proximal end (33) of the cartridge (3) in a radial rotational movement; and
- a step d) of actuating the actuating element (42) of the core (41) of the plunger (4), so that the core (41) is moved along its longitudinal axis in the guiding channel (49) of the outer sleeve (43) and relative to the outer sleeve (43) and thereby the engaged stopper (33) at the proximal end (33) of the cartridge (3) is pushed into the ampule cylinder (32).

15. Use of the cartridge syringe (1) according to claims 1 to 12 for applying a drug, a medicament or another formulation to a subject.
